(19) Europäisches Patentamt
European Patent Office
Office européen des brevets

(11) EP 1 797 882 A1

(12) **EUROPEAN PATENT APPLICATION**
published in accordance with Art. 158(3) EPC

(43) Date of publication:
**20.06.2007 Bulletin 2007/25**

(51) Int Cl.:
**A61K 31/519** (2006.01)    **A61K 31/50** (2006.01)
**A61P 29/00** (2006.01)

(21) Application number: **05787991.8**

(22) Date of filing: **29.09.2005**

(86) International application number:
**PCT/JP2005/017959**

(87) International publication number:
**WO 2006/035876 (06.04.2006 Gazette 2006/14)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **29.09.2004 US 613716 P**

(71) Applicant: **Kowa Co., Ltd.**
**Nagoya-shi, Aichi 460-8625 (JP)**

(72) Inventors:
• **TABUNOKI, Yuichiro**
**2030051 (JP)**
• **KOSHI, Tomoyuki**
**3530006 (JP)**

(74) Representative: **Hartz, Nikolai et al**
**Wächtershauser & Hartz**
**Weinstrasse 8**
**80333 München (DE)**

(54) **PREVENTIVE AND/OR THERAPEUTIC MEDICINE FOR RHEUMATOID ARTHRITIS**

(57)    The present invention relates to a preventive and/or therapeutic medicine for rheumatoid arthritis, containing 2-benzyl-5-(4-chlorophenyl)-6-[4-(methylthio) phenyl]-2H-pyridazin-3-one and methotrexate. The medicine of the present invention can be administered orally and exhibits suppressed side effects and excellent potency for suppression of arthritis.

Fig. 1

Drug A: 2-Benzyl-5-(4-chlorophenyl)-6-[4-(methylthio)phenyl]-2H-pyridazin-3-one

Drug B: Methotrexate

EP 1 797 882 A1

## Description

Technical Field

The present invention relates to a medicine for the prevention and/or treatment of rheumatoid arthritis.

Background Art

Rheumatoid arthritis is a disease which involves inflammation in many joints, concomitant with swelling and pain. When rheumatoid arthritis has progressed for a long period of time, the patient suffers irreversible deformity in the joints and functional disorders, and the quality of life (QOL) of the patient is deteriorated considerably. In Japan, 0.6% of the total population and 1% of the population over age 30 suffer rheumatoid arthritis. With the progressive aging of society in recent years, elderly patients suffering from rheumatoid arthritis have gradually increased in number.

Rheumatoid arthritis progresses through the following four stages. In the initial stage, joint pain and arthritis are observed, but the patient cannot be definitely diagnosed as suffering from rheumatoid arthritis. In the early stage, the patient can be definitely diagnosed as suffering from rheumatoid arthritis, but exhibits no or slight irreversible deformity (early stage of rheumatoid arthritis generally refers to a stage after 1 to 2 years from the onset of the disease). In the progressive stage, the patient exhibits irreversible deformity and significant systemic symptoms, including fatigue, low-grade fever, and weight loss. In the late stage, arthritis is almost sedated, but irreversible deformity such as deformity/contracture remains, resulting in pain and functional disorders as predominant symptoms. The method for the treatment varies depending on the stage of rheumatoid arthritis. The onset mechanism of rheumatoid arthritis has not yet been elucidated, although some studies have reported a correlation between the onset of the disease and a hereditary factor or an acquired factor (i.e., an infectious disease). Since the cause of the disease has not been clarified, complete prevention and curing of rheumatoid arthritis is still not possible.

Thus, at present, goals of rheumatoid arthritis treatment are early establishment of diagnosis as rheumatoid arthritis and suppressing inflammation caused by rheumatoid arthritis as soon as and as effectively as possible, whereby expression or progress of irreversible deformity is prevented so as to enhance QOL of patients from physical, mental, and social aspects. In this connection, upon the treatment of rheumatoid arthritis, the patients are well instructed in advance in terms of characteristics and the treatment of the disease, and receive a variety of treatment means such as physical therapy, kinesitherapy, drug therapy, and surgery.

In drug therapy, drugs such as non-steroidal antiinflammatory drugs (NSAIDs), disease-modifying anti-rheumatic drugs (DMARDs), and corticosteroids are employed in clinical settings. Recently, biologics such as antibodies against proinflammatory cytokines are also employed (Non-Patent Document 1).

DMARDs are categorized, in terms of the mechanism of action, into an immunomodulator and an immunosuppressant. Among DMARDs, methotrexate (N-[4-[(2,4-diamino-6-pteridinyl)methylamino]benzoyl]-L-glutamic acid), which is an antifolic immunosuppressant (dihydrofolic acid reductase inhibitory action), is recognized to exhibit a high effect of suppressing rheumatoid arthritis. However, since the efficacy range of methotrexate has been extended from its original efficacy as an anti-cancer drug, detrimental side effects such as interstitial pneumonia are induced. Therefore, use of methotrexate accompanies a drawback such that mode of administration and dosage must be strictly controlled.

The aforementioned biologics exhibit potent antirheumatism effects. However, they accompany drawbacks such that they are expensive (i.e., disadvantage in medical cost) and that they can be administered only through injection and cannot be perorally administered (i.e., involves a disadvantage in handling).

In many diseases such as rheumatoid arthritis, osteoarthritis, osteoporosis, inflammatory bowel disease, immune deficiency syndrome, septicemia, hepatitis, nephritis, ischemic diseases, insulin-dependent diabetes, arteriosclerosis, Perkinson's disease, Alzheimer's disease, and leukemia, stimulation of production of interleukin 1β (IL-1β), which is an inflammatory cytokine, is observed. IL-1β is known to induce synthesis of enzymes which are conceived to involve inflammation; e.g., collagenase, COX, and PLA2, and to cause articular damage very similar to that caused by rheumatoid arthritis when intra-articularly injected to animals. Thus, IL-1β inhibitors are studied and developed to serve as drugs for the treatment of inflammatory diseases. Specifically, hitherto, there have been known a bio-substance such as IL-1 receptor antagonist (Non-Patent Document 2), a low-molecular-weight compound such as T-614 (Non-Patent Document 3), S-2474 (Non-Patent Document 4), 2-benzyl-5-(4-chlorophenyl)-6-[4-(methylthio)phenyl]-2H-pyridazin-3-one (Patent Document 1), and FR133605 (Non-Patent Document 5).

Pathological conditions of rheumatoid arthritis vary considerably among patients in terms of age, stage, complications, side effects, QOL, etc. Hitherto, an ultimate therapeutic drug therefore has not yet been developed. Even though a therapeutic drug can control the disease condition, in some cases, the effect of the drug is suddenly lost, which is called "escape phenomenon." Under such circumstances, when drug therapy is performed, change of drugs and combined use of drugs are generally employed. Thus, clinical studies on the mode of use of such drugs are carried out extensively.

Among the combined effects of an antifolic and IL-1β inhibitor, the effect of combined use of methotrexate and IL-1 inhibitor has already been reported (Patent Documents 2 and 3, and Non-Patent Document 6).

However, the aforementioned IL-1 inhibitor is a human recombinant IL-1 receptor antagonist; i.e., one of the biologics. Therefore, the drug form thereof is limited to an injection, which is not satisfactory for patients in term of convenience. In addition, administration of the drug may induce rejection reaction. Therefore, there has been demand for a method for preventing/treating rheumatoid arthritis, which can take a form of peroral administration and exhibit suppressed side effects.

Meanwhile, hitherto, nothing has been known about the effect of combined use of an antifolic and a low-molecular-weight compound exhibiting the aforementioned IL-1β inhibitory action.

[Patent Document 1] WO99/25697
[Patent Document 2] JP-A-2002-509529
[Patent Document 3] US2004/0044001
[Non-Patent Document 1] American College of Rheumatology Subcommittee on Rheumatoid Arthritis Guidelines, Arthritis & Rheumatism 46, pp 328-346, 2002
[Non-Patent Document 2] Arthritis & Rheumatism 42, pp 498-506, 1999
[Non-Patent Document 3] J. Pharmacobio-Dyn. 11, pp 649-655, 1992
[Non-Patent Document 4] YAKUGAKU ZASSHI 123, pp 323-330, 2003
[Non-Patent Document 5] J. Rheumatol. 23, pp 1778-1783, 1996
[Non-Patent Document 6] J. Rheumatol. 26, pp 1225-1229, 1999

Disclosure of the Invention

In view of the foregoing, the present inventors have conducted extensive studies, and have found that use in combination of 2-benzyl-5-(4-chlorophenyl)-6-[4-(methylthio)phenyl]-2H-pyridazin-3-one, which is an IL-1β inhibitor, and methotrexate, which is an antifolic, results in an excellent effect of preventing arthritis. The present invention has been accomplished on the basis of this finding.

Accordingly, the present invention provides a preventive and/or therapeutic medicine for rheumatoid arthritis, characterized by comprising 2-benzyl-5-(4-chlorophenyl)-6-[4-(methylthio)phenyl]-2H-pyridazin-3-one and methotrexate.

The present invention also provides a method for the prevention and/or treatment of rheumatoid arthritis, characterized in that the method comprises administering 2-benzyl-5-(4-chlorophenyl)-6-[4-(methylthio)phenyl]-2H-pyridazin-3-one and methotrexate.

The present invention also provides use of 2-benzyl-5-(4-chlorophenyl)-6-[4-(methylthio)phenyl]-2H-pyridazin-3-one and methotrexate for production of a preventive and/or therapeutic medicine for rheumatoid arthritis.

Since the medicine according to the present invention can be administered orally and exhibits suppressed side effects and excellent potency for suppression of arthritis, the medicine is useful for the prevention and/or treatment of rheumatoid arthritis.

Brief Description of the Drawing

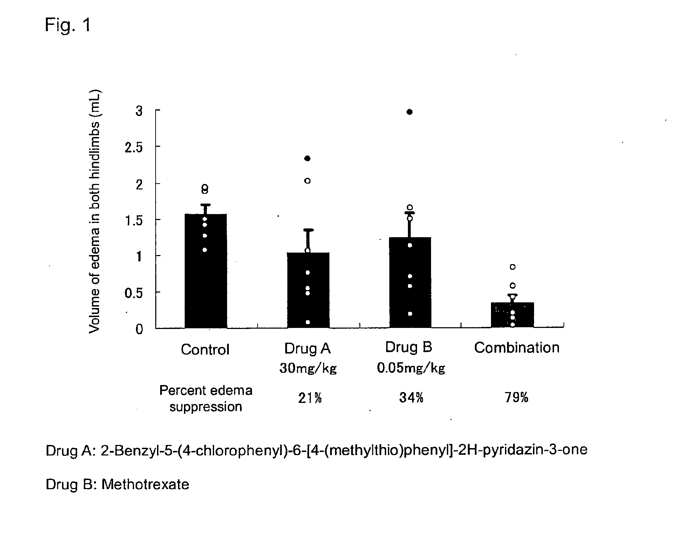

[Fig. 1] A graph showing the volume of edema produced in both hindlimbs of rats of a collagen-induced arthritis model measured 30 days after initial sensitization, where the rats were divided into a control group (drug-free group), a drug A-administration group (i.e., 2-benzyl-5-(4-chlorophenyl)-6-[4-(methylthio)phenyl]-2H-pyrridazin-3-one, 30 mg/kg), a drug B-administration group (i.e., methotrexate, 0.05 mg/kg), and a combined administration group (drugs A and B)). In the graph, the data of rats that were conceived to exhibit the escape phenomenon are indicated with closed circles.

Best Modes for Carrying Out the invention

2-Benzyl-5-(4-chlorophenyl)-6-[4-(methylthio)phenyl]-2H-pyridazin-3-one employed in the present invention may be produced through the method disclosed in WO 99/25697 or a similar method. Specifically, p-chlorophenylacetic acid is reacted with thioanisole in the presence of a condensing agent such as polyphosphoric acid, to thereby form 2-(4-chlorophenyl)-4'-(methylthio)acetophenone. The thus-formed 2-(4-chlorophenyl)-4'-(methylthio)acetophenone is reacted with a base such as potassium t-butoxide in tetrahydrofuran, followed by addition of ethyl bromoacetate to the reaction system, to thereby form ethyl 2-(4-chlorophenyl)-4-[4-(methylthio)phenyl]-4-oxobutanate. The thus-formed ethyl 2-(4-chlorophenyl)-4-[4-(methylthio)phenyl]-4-oxobutanate is reacted with hydrazine hydrate in ethanol, to thereby form 5-(4-chlorophenyl)-6-[4-(methylthio)phenyl]-4,5-dihydro-2H-pyridazin-3-one. The thus-formed 5-(4-chlorophenyl)-6-[4-(methylthio)phenyl]-4,5-dihydro-2H-pyridazin-3-one is reacted with benzyl bromide in a solvent such as N,N-dimethylformamide in the presence of a base such as potassium carbonate, to thereby yield 2-benzyl-5-(4-chlorophenyl)-6-[4-(methylthio)phenyl]-2H-pyridazin-3-one.

The antifolic employed in the present invention is preferably methotrexate. A commercial product of methotrexate such as a product of SIGMA may be used.

When 2-benzyl-5-(4-chlorophenyl)-6-[4-(methylthio)phenyl]-2H-pyridazin-3-one and methotrexate are used in combination, a remarkable therapeutic effect for rheumatoid arthritis can be attained, as compared with the case where these compounds are used singly.

2-Benzyl-5-(4-chlorophenyl)-6-[4-(methylthio)phenyl]-2H-pyridazin-3-one and methotrexate are preferably employed at a ratio by mass of 1000 : 1 to 1: 1.

2-benzyl-5-(4-chlorophenyl)-6-[4-(methylthio)phenyl]-2H-pyridazin-3-one and methotrexate may be orally administered simultaneously or at a predetermined interval, or else, they may be orally administered as a combination drug.

No particular limitation is imposed on the mode of administering the medicine of the present invention, and the mode may be appropriately selected in accordance with the purpose of the treatment. In oral administration, tablets, capsules, granules, film-coated drugs, powders, and syrups may be employed. In parenteral administration, injections, suppositories, inhalations, percutaneous drugs, eye drops, and nasal drops may be employed. Of these, oral administration is preferred.

The pharmaceutical preparation suited for the above modes of administration may appropriately be employed with the following additives: pharmacologically acceptable carriers (vehicles and bulking agents) such as starch, lactose, sucrose, mannitol, and silicic acid; disintegrants such as agar, calcium carbonate, potato or tapioca starch, alginic acid, and specific complex silicate salts; binders such as hydroxypropyl methyl cellulose, alginate salts, gelatin, polyvinylpyrrolidone, sucrose, and acacia; lubricants such as talc, calcium stearate, magnesium stearate, solid polyethylene glycols, sodium lauryl sulfate, and mixtures thereof; diluents such as lactose and corn starch; buffers such as organic acids (e.g., citric acid, phosphoric acid, tartaric acid, and lactic acid), inorganic acids (e.g., hydrochloric acid), alkali hydroxides (e.g., sodium hydroxide and potassium hydroxide), and amines (e.g., triethanolamine, diethanolamine, and diisoproanolamine); antiseptic agents such as p-oxybenzoate esters and benzalkonium chloride; emulsifying agents such as anionic surfactants (e.g., calcium stearate, magnesium stearate, and sodium lauryl sulfate), cationic surfactants (e.g., benzalkonium chloride, benzetonium chloride, and cetylpyridinium chloride), and nonionic surfactants (e.g., glyceryl monostearate, sucrose fatty acid esters, polyoxyethylene-hydrogenated castor oil, polyoxyethylene sorbitan fatty acid esters, polyoxyethylene fatty acid esters, and polyoxyethylene alkyl ethers); and stabilizing agents such as sodium sulfite, sodium bisulfite, dibutylhydroxytoluene, butylhydroxyanisole, and EDTA. In addition, additives such as an odor-suppressor, a dispersant, a preservative, and a flavoring may appropriately be used in accordance with needs.

In the present invention, 2-benzyl-5-(4-chlorophenyl)-6-[4-(methylthio)phenyl]-2H-pyridazin-3-one and methotrexate may be administered in a single dose per day, or in two or more doses per day in a divided manner.

Examples

The present invention will next be described in detail by way of examples, which should not be construed as limiting the invention thereto.

Example 1

The effect on suppressing edema of both hindlimbs by 2-benzyl-5-(4-chlorophenyl)-6-[4-(methylthio)phenyl]-2H-pyridazin-3-one (synthesized through the aforementioned method) and methotrexate were determined both in the cases of combined administration and solo administration through the following procedure. For the experiment, rats of a collagen-induced arthritis model were used. (FDA, CBER, CDER, CDRH: Guidance for industry - Clinical development programs for drugs, devices, and biological products for the treatment of rheumatoid arthritis (RA)-.(1999)). Lewis female rats (LEW/Crj) (obtained from Charles River Laboratories Japan, Inc.) were employed as test animals.

For each 8-week-old LEW/Crj rat, the volume of a portion from the ankle to the toe tip of each hindlimb was measured by means of a plethysmometer for small animals (TK-101CMP, product of Unicom), and the total volume was employed as a volume of the hindlimbs (hereinafter referred to as both-hindlimb volume) at the start of the test (hereinafter referred to as Pre value). By use of the Pre value as an index, the rats were divided into groups by one-parameter-based block randomization so that the values in the groups were averaged out.

The collagen emulsion for sensitization employed for inducing arthritis in rats was prepared by homogenizing a 0.3% Type II collagen liquid (product of Collagen Research Center), adjuvant peptide (product of Peptide Institute, Inc.), and adjuvant incomplete Freund (product of DIFCO) by means of a Handy Micro Homogenizer (product of Microtec Nition) under cooling with ice. The thus-prepared emulsion was intracutaneously injected to 10 sites in the back of each rat at 0.1 mL/site, to thereby initially sensitize the rat. Seven days after initial sensitization, the same emulsion (0.12 mL) was intracutaneously injected to the tail head of the rat for booster sensitization.

Administration of medicines was carried out on the day after initial sensitization to day 29. To the 2-benzyl-5-(4-chlorophenyl)-6-[4-(methylthio)phenyl]-2H-pyridazin-3-one solo administration group, the medicine was orally administered twice; in the morning (9:00 to 11:00) and in the evening (15:30 to 17:30) at a dose of 30 mg/kg. To the methotrexate sole administration group, the medicine was orally administered once in the afternoon (11:30 to 13:30) at a dose of 0.05 mg/kg. To the combined administration group (2-benzyl-5-(4-chlorophenyl)-6-[4-(methylthio)phenyl]-2H-pyridazin-3-one and methotrexate), 2-benzyl-5-(4-chlorophenyl)-6-[4-(methylthio)phenyl]-2H-pyridazin-3-one was orally administered in the morning (9:00 to 11:00) and in the evening (15:30 to 17:30) at a dose of 30 mg/kg, and methotrexate was orally administered in the afternoon (11:30 to 13:30) at a dose of 0.05 mg/kg.

Thirty days after initial sensitization, both-hindlimb volume was measured. Volume of both-hindlimb edema was calculated by subtracting the Pre value from the thus-measured value.

Table 1 and Fig. 1 show both-hindlimb edema volume of rats which was measured 30 days after initial sensitization, where the rats were divided into the 2-benzyl-5-(4-chlorophenyl)-6-[4-(methylthio)phenyl]-2H-pyridazin-3-one solo administration group, the methotrexate solo administration group, and the combined administration group. The volume of both-hindlimb edema (mL) of each group is an average of the volumes observed in seven rats $\pm$ a standard error. Percent edema suppression represents a value obtained from the following formula:

$$100 \times [((\text{average both-hindlimb edema volume of the control group}) - (\text{average both-hindlimb edema volume of each administration group})) / (\text{average both-hindlimb edema volume of the control group})].$$

Relative index is expressed by the following formula:

$$(\text{average both-hindlimb edema volume of each administration group}) / (\text{average both-hindlimb edema volume of the control group}).$$

As is clear from Table 1 and Fig. 1, solo administration of 2-benzyl-5-(4-chlorophenyl)-6-[4-(methylthio)phenyl]-2H-pyridazin-3-one and that of methotrexate did not result in potent edema suppression effects. Actually, in both cases, the edema volume did not decrease to 50% or less with respect to that of the control group.

In contrast, combined administration of both medicines exhibits a potent effect on reducing both-hindlimb edema volume. The relative index (0.21) was smaller than the product (0.52) of the relative index of the solo administration groups, indicating that a clear synergistic effect could be attained through combined administration. In addition, the prevention of the escape phenomenon was made possible.

[Table 1]

| Tested medicines | Both-hindlimb edema volume (mL) | Percent suppression (%) | Relative index |
|---|---|---|---|
| Control group | 1.57±0.13 | | |
| 2-Benzyl-5-(4-chlorophenyl)-6-[4-(methylthio)phenyl]-2H-pyridazin-3-one solo administration group (30 mg/kg) | 1.04+0.32 | 34 | 0.66 |
| Methotrexate solo administration group (0.05 mg/kg) | 1.24±0.35 | 21 | 0.79 |

(continued)

| Tested medicines | Both-hindlimb edema volume (mL) | Percent suppression (%) | Relative index |
|---|---|---|---|
| Combined administration group | 0.33±0.11 | 79 | 0.21 |
| Both-hindlimb edema volume of each group is an average of the volumes observed in seven rats ± a standard error. | | | |

**Claims**

1. A preventive and/or therapeutic medicine for rheumatoid arthritis, comprising 2-benzyl-5-(4-chlorophenyl)-6-[4-(methylthio)phenyl]-2H-pyridazin-3-one and methotrexate.

2. A preventive and/or therapeutic medicine for rheumatoid arthritis as described in claim 1, wherein the rheumatoid arthritis involves inflammation of a joint.

3. A preventive and/or therapeutic medicine for rheumatoid arthritis as described in claim 1 or 2, wherein the medicine is a peroral administration preparation.

4. Use of 2-benzyl-5-(4-chlorophenyl)-6-[4-(methylthio)phenyl]-2H-pyridazin-3-one and methotrexate for production of a preventive and/or therapeutic medicine for rheumatoid arthritis.

5. Use as described in claim 4, wherein the rheumatoid arthritis involves inflammation of a joint.

6. Use as described in claim 4 or 5, wherein the medicine is a peroral administration preparation.

7. A method for the prevention and/or treatment of rheumatoid arthritis, **characterized in that** the method comprises administering 2-benzyl-5-(4-chlorophenyl)-6-[4-(methylthio)phenyl]-2H-pyridazin-3-one and methotrexate.

8. A method for the prevention and/or treatment of rheumatoid arthritis as described in claim 7, wherein the rheumatoid arthritis involves inflammation of a joint.

9. A method for the prevention and/or treatment of rheumatoid arthritis as described in claim 7 or 8, wherein the administration means is peroral administration.

Fig. 1

| Percent edema suppression | 21% | 34% | 79% |

Drug A: 2-Benzyl-5-(4-chlorophenyl)-6-[4-(methylthio)phenyl]-2H-pyridazin-3-one

Drug B: Methotrexate

**INTERNATIONAL SEARCH REPORT**

| International application No. |
| --- |
| PCT/JP2005/017959 |

A. CLASSIFICATION OF SUBJECT MATTER
*A61K31/519*(2006.01), *A61K31/50*(2006.01), *A61P29/00*(2006.01)

According to International Patent Classification (IPC) or to both national classification and IPC

B. FIELDS SEARCHED

Minimum documentation searched (classification system followed by classification symbols)
*A61K31/519*(2006.01), *A61K31/50*(2006.01)

Documentation searched other than minimum documentation to the extent that such documents are included in the fields searched
| | | | |
| --- | --- | --- | --- |
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
REGISTRY(STN), CAPLUS(STN), MEDLINE(STN), BIOSIS(STN), EMBASE(STN)

C. DOCUMENTS CONSIDERED TO BE RELEVANT

| Category* | Citation of document, with indication, where appropriate, of the relevant passages | Relevant to claim No. |
| --- | --- | --- |
| Y | JP 2002-509529 A (Amjen Inc.), 26 March, 2002 (26.03.02), Full text; particularly, Claims; page 7, lines 1 to 4; page 8, lines 7 to 20; page 59, lines 10 to 23; examples & WO 98/24477 A1 & EP 949931 A1 & US 2003/072756 A1 & CA 2273852 A & MX 9905227 A | 1-6 |
| Y | WO 99/25697 A1 (Kowa Co., Ltd.), 27 May, 1999 (27.05.99), Full text; particularly, Claims; example 51; test examples 1 to 3 & JP 11-152274 A & EP 1043317 A1 & US 2002/123496 A1 & CA 2307111 A & BR 9813998 A | 1-6 |

☐ Further documents are listed in the continuation of Box C.     ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>17 October, 2005 (17.10.05) | Date of mailing of the international search report<br>20 December, 2005 (20.12.05) |
| --- | --- |
| Name and mailing address of the ISA/<br>Japanese Patent Office | Authorized officer |
| Facsimile No. | Telephone No. |

Form PCT/ISA/210 (second sheet) (April 2005)

## INTERNATIONAL SEARCH REPORT

| | International application No. |
|---|---|
| | PCT/JP2005/017959 |

**Box No. II       Observations where certain claims were found unsearchable (Continuation of item 2 of first sheet)**

This international search report has not been established in respect of certain claims under Article 17(2)(a) for the following reasons:

1. ☒ Claims Nos.: 7-9
   because they relate to subject matter not required to be searched by this Authority, namely:
   Claims 7 to 9 pertain to methods for treatment of the human body by surgery or therapy and thus relate to a subject matter which this International Searching Authority is not required to search.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III       Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**        ☐ The additional search fees were accompanied by the applicant's protest and, where applicable, the payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 133605
- WO 9925697 A
- JP 2002509529 A
- US 20040044001 A

**Non-patent literature cited in the description**

- American College of Rheumatology Subcommittee on Rheumatoid Arthritis Guidelines. *Arthritis & Rheumatism,* 2002, vol. 46, 328-346
- *Arthritis & Rheumatism,* 1999, vol. 42, 498-506
- *J. Pharmacobio-Dyn.,* 1992, vol. 11, 649-655
- *YAKUGAKU ZASSHI,* 2003, vol. 123, 323-330
- *J. Rheumatol.,* 1996, vol. 23, 1778-1783
- *J. Rheumatol.,* 1999, vol. 26, 1225-1229